# EUROPEAN PATENT SPECIFICATION

(11) **EP 3 606 607 B1**
(45) Date of publication and mention of the grant of the patent: **17.03.2021**
(21) Application number: 18718354.6
(22) Date of filing: 29.03.2018
(51) Int. Cl.: A61N 1/378

(54) **IMPLANTABLE MEDICAL DEVICE WITH A TETHERED TRANSMIT COIL FOR TRANSMITTING POWER TO ANOTHER IMPLANTABLE MEDICAL DEVICE**
IMPLANTIERBARE MEDIZINISCHE VORRICHTUNG MIT EINER ANGEBUNDENEN SENDESPULE ZUR ÜBERTRAGUNG VON STROM AN EINE ANDERE IMPLANTIERBARE MEDIZINISCHE VORRICHTUNG
DISPOSITIF MÉDICAL IMPLANTABLE AVEC BOBINE D'ÉMISSION ATTACHÉE POUR TRANSMETTRE DE L'ÉNERGIE À UN AUTRE DISPOSITIF MÉDICAL IMPLANTABLE

(30) Priority: 03.04.2017 US 201762480753 P
(43) Date of publication of application: 12.02.2020
(73) Proprietor: Cardiac Pacemakers, Inc., St. Paul, Minnesota 55112 (US)
(72) Inventor: MAILE, Keith R., New Brighton, Minnesota 55112 (US); LUDWIG, Jacob M., Isanti, Minnesota 55040 (US); JOHNSON, Michael J., North Oaks, Minnesota 55127 (US); KOOP, Brendan Early, Ham Lake, Minnesota 55304 (US); FELLOWS, Brandon Christopher, Chicago, Illinois 60611 (US); LINDER, William J., Golden Valley, Minnesota 55422 (US)
(74) Representative: Pfenning, Meinig & Partner mbB
(86) International application number: PCT/US2018/025199
(87) International publication number: WO 2018/187157

(56) References cited:
- WO-A2-2013/121290
- US-A1- 2007 170 887
- US-A1- 2012 146 575
- US-B1- 9 216 285

## Description

### TECHNICAL FIELD

The present disclosure pertains to medical devices, and more particularly to implantable medical devices that are capable of delivering power to another implantable medical device.

### BACKGROUND

Implantable medical devices are commonly used today to monitor physiological or other parameters of a patient and/or deliver therapy to a patient. In one example, to help patients with heart related conditions, various medical devices (e.g., pacemakers, defibrillators, etc.) can be implanted in a patient's body. Such devices may monitor and in some cases provide electrical stimulation (e.g. pacing, defibrillation, etc.) to the heart to help the heart operate in a more normal, efficient and/or safe manner. In another example, neuro stimulators can be used to stimulate tissue of a patient to help alleviate pain and/or other condition. In yet another example, an implantable medical device may simply be an implantable monitor that monitors one or more physiological or other parameters of the patient, and communicates the sensed parameters to another device such as another implanted medical device or an external device. In some cases, two or more implantable medical devices are implanted within the same patient, sometimes working together to monitor and/or treat one or more conditions of the patient. The desired placement of the implantable medical devices within the patient's body can imposed size constraints on the implantable medical devices. Some implantable medical devices may have significant size constraints, while others may be given more latitude. The implantable medical devices with significant size constraints may have less room for a power supply such as a battery. To help implantable medical devices with smaller power storage capacity, it can be desirable to periodically or intermittently deliver power to the implantable medical device, such as from another implantable medical device.

US 2007 170887 A1 discusses a system for charging a rechargeable battery in one implantable device using a charger located in a second implantable device.

US 9216285 B1 discusses a leadless implantable medical device including an electrode, a housing, and an energy transfer component. The energy transfer component is distributed between the first and second body portions and is configured to convey at least one of stimulation energy or sensed signals across the detachable interface when first and second body portions are mated to one another.

### SUMMARY

The present disclosure pertains to medical devices, and more particularly to implantable medical devices (IMDs) that are capable of delivering power to another implantable medical device. The IMDs may include, for example, leadless cardiac pacemakers (LCP), subcutaneous implantable cardioverter defibrillators (SICD), transvenous implantable cardioverter defibrillators, neuro-stimulators (NS), implantable monitors (IM), and/or the like.

The invention is defined by the appended claims.

### BRIEF DESCRIPTION OF THE DRAWINGS

The disclosure may be more completely understood in consideration of the following description in connection with the accompanying drawings, in which:
Figure 1 is a schematic block diagram of a system including a first implantable medical device (IMD) and a second implantable medical device (IMD) in accordance with an example of the disclosure;
Figure 2 is a schematic block diagram of a system including an implantable cardioverter-defibrillator (ICD) and a remote implantable medical device (IMD) in accordance with an example of the disclosure;
Figure 3 is a schematic block diagram of an implantable device in accordance with an example of the disclosure;
Figure 4 is a schematic block diagram of an implantable medical device (IMD) in accordance with an example of the disclosure;
Figure 5A and 5B are schematic block diagrams of power supplies that may be used as part of the IMD of Figure 4;
Figure 6 is a schematic block diagram of an implantable cardioverter-defibrillator (ICD) in accordance with an example of the disclosure;
Figure 7 is a schematic block diagram of an implantable medical device (IMD) in accordance with an example of the disclosure;
Figure 8 is a more detailed schematic block diagram of an illustrative IMD in accordance with an example of the disclosure;
Figure 9 is a schematic block diagram of another illustrative medical device that may be used in conjunction with the IMD of Figure 8;
Figure 10 is a schematic diagram of an illustrative medical system that includes multiple LCPs and/or other devices in communication with one another; and
Figure 11 is a schematic diagram of a system including an LCP and another medical device, in accordance with an example of the disclosure.

While the disclosure is amenable to various modifications and alternative forms, specifics thereof have been shown by way of example in the drawings and will be described in detail.

### DESCRIPTION

For the following defined terms, these definitions shall be applied, unless a different definition is given in the claims or elsewhere in this specification.

All numeric values are herein assumed to be modified by the term "about," whether or not explicitly indicated. The term "about" generally refers to a range of numbers that one of skill in the art would consider equivalent to the recited value (i.e., having the same function or result). In many instances, the terms "about" may include numbers that are rounded to the nearest significant figure.

The recitation of numerical ranges by endpoints includes all numbers within that range (e.g. 1 to 5 includes 1, 1.5, 2, 2.75, 3, 3.80, 4, and 5).

As used in this specification and the appended claims, the singular forms "a", "an", and "the" include plural referents unless the content clearly dictates otherwise. As used in this specification and the appended claims, the term "or" is generally employed in its sense including "and/or" unless the content clearly dictates otherwise.

It is noted that references in the specification to "an embodiment", "some embodiments", "other embodiments", etc., indicate that the embodiment described may include one or more particular features, structures, and/or characteristics. However, such recitations do not necessarily mean that all embodiments include the particular features, structures, and/or characteristics. Additionally, when particular features, structures, and/or characteristics are described in connection with one embodiment, it should be understood that such features, structures, and/or characteristics may also be used connection with other embodiments whether or not explicitly described unless clearly stated to the contrary.

The following detailed description should be read with reference to the drawings in which similar structures in different drawings are numbered the same. The drawings, which are not necessarily to scale, depict illustrative embodiments and are not intended to limit the scope of the disclosure. While the present disclosure is applicable to any suitable implantable medical device (IMD), the description below often uses implantable cardioverter-defibrillator (ICD) and/or pacemakers as particular examples.

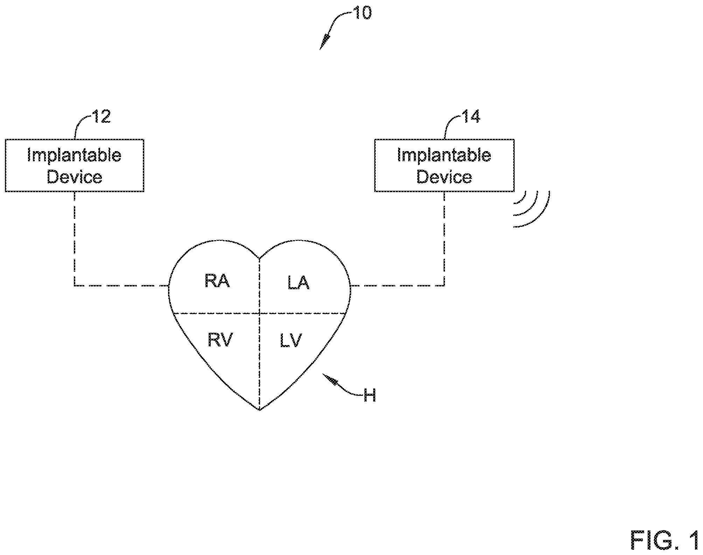

Figure 1 is a schematic diagram showing an illustrative system 10 that may be used to sense and/or pace a heart H. In some cases, the system 10 may also be configured to shock the heart H. The heart H includes a right atrium RA and a right ventricle RV. The heart H also includes a left atrium LA and a left ventricle LV. In some cases, the system 10 may include a medical device that provides anti-arrhythmic therapy to the heart H. In some cases, the system 10 may include a first implantable medical device (IMD) 12 and a second implantable medical device (IMD) 14. In some instances, the first IMD 12 and the second IMD 14 may each be implantable within the patient at a position near or even within the heart H. In some cases, the first IMD 12 may be implanted away from the heart H while the second IMD 14 is implanted within the heart H.

In some instances, as will be discussed, the first IMD 12 may be configured to at least partially power the second IMD 14 by periodically, intermittently or continuously recharging a rechargeable battery or other power source within the second IMD 14. In some cases, as will be discussed, the first IMD 12 may at least partially power the second IMD 14 by periodically, intermittently or continuously transmitting energy that may be stored by a capacitor or other power storage device within the second IMD 14. In some cases, the first IMD 12 may be configured to provide therapy to the heart H. For example, in some cases, the first IMD 12 may be an implantable cardioverter-defibrillator (ICD) or an implantable subcutaneous cardioverter-defibrillator (SICD). In some cases, the first IMD 12 may include a power supply and a transmitter coil for inductively transmitting power to the second IMD 14, but may not itself provide therapy to the heart H. In some cases, the second IMD 14 may be a leadless cardiac pacemaker (LCP) that has a receive coil, for example. In some cases, for example, the first IMD 12 may itself be configured to receive power that is transmitted inductively from a power source exterior to the patient. The first IMD 12 may receive power and then transmit the received power to the second IMD 14. In some cases, the first IMD 12 and the second IMD 14 may both receive power from the power source exterior to the patient. While the transmission and reception of inductive energy is described, in some cases RF and/or acoustic energy may also be used.

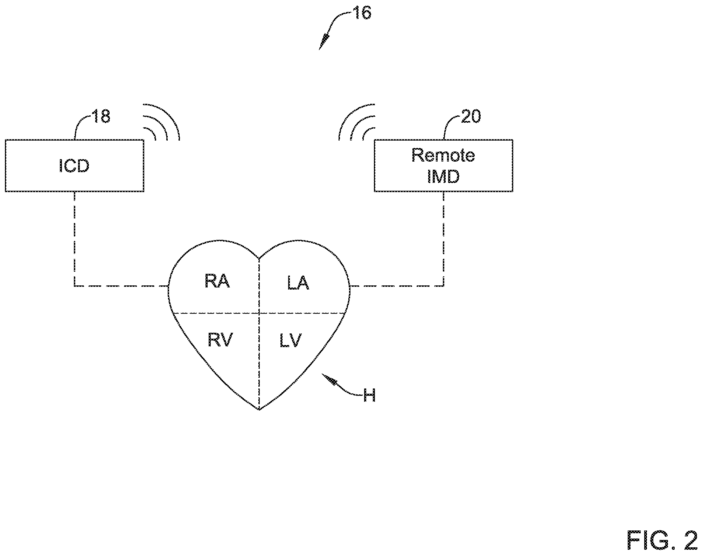

Figure 2 is a schematic diagram showing an illustrative system 16 that may be used to sense and/or pace a heart H. In some cases, the system 16 may also be configured to shock the heart H. In some cases, the system 16 may include an implantable cardioverter-defibrillator (ICD) 18 and an implantable medical device (IMD) 20. In some cases, as will be discussed, the ICD 18 may be configured to at least partially power the IMD 20 by periodically, intermittently or continuously recharging a rechargeable battery or other power source (e.g. capacitor) within the IMD 20. In some cases, as will be discussed, the ICD 18 may at least partially power the IMD 20 by periodically, intermittently or continuously transmitting energy that may be stored by a capacitor or other power storage device within the second IMD 14. In some cases, the IMD 20 may be a leadless cardiac pacemaker (LCP) with a receive coil, for example.

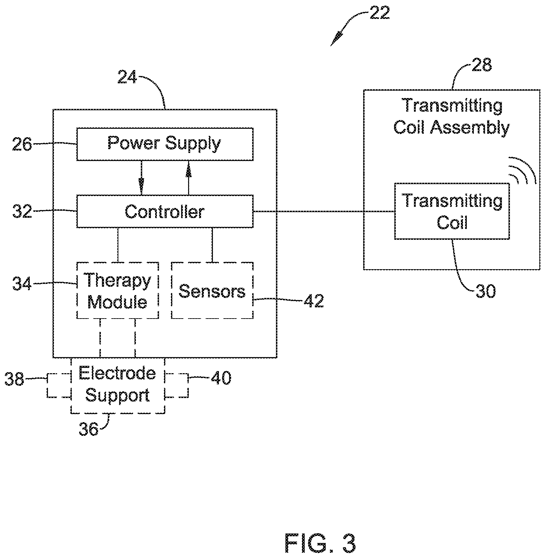

Figure 3 is a schematic block diagram of an implantable device 22 that may, for example, be considered as being an example of the first IMD 12 (Figure 1). In some cases, for the example, the implantable device 22 may be an implantable cardioverter-defibrillator (ICD) or an implantable subcutaneous cardioverter-defibrillator (SICD). In some instances, the implantable device 22 may be one or more of a neurostimulation device and a drug delivery device. In the example shown, the implantable device 22 includes a housing 24 that is configured to be implantable at a subcutaneous implant location within a patient. The implantable device 22 includes a power supply 26 that is disposed within the housing 24 as well as a transmitting coil assembly 28 that extends from the housing 24.

While shown schematically, it will be appreciated that the transmitting coil assembly 28 may be configured to extend from the subcutaneous implant location of the housing 24 to a substernal location. The transmitting coil assembly 28 may tether a transmit coil 30 to the housing 24. In some cases, the transmitting coil assembly 28 may be similar to an elongated lead with a transmit coil mounted at or near the distal end. The transmitting coil assembly 28 may be flexible so that a physician can place the transmit coil 30 at a desired location, such as a substernal location adjacent a second IMD 14 that is implanted within or on the heart. The transmitting coil assembly 28 may have a length of 1 inches, 3 inches, 4 inches, 6 inches, 8 inches, 12 inches or more, depending on the application.

In the example shown, a controller 32 is operably coupled to the power supply 26 and to the transmit coil 30. The controller 32 is configured to selectively activate the transmit coil 30 to provide inductive power to a receive coil of a remote medical device implanted substernal (e.g. in or on the heart). The second IMD 14 (Figure 1) may be considered as being an example of such a remote medical device including a receive coil.

In some cases, the implantable device 22 may be configured to provide power to a remote device but may not itself be configured to sense cardiac signals such as electrical cardiac signals and/or to provide therapy such as pacing or shock therapy to the heart H. In some cases, the implantable device 22 may be configured to have additional functionality beyond providing power. For example, in some cases, as illustrated, the implantable device 22 may include a therapy module 34 that is operably coupled to the controller 32 for generating a therapy. In some cases, the implantable device 22 may include an electrode support 36 that extends from the housing 24 and that supports one or more electrodes. As illustrated, the electrode support 36 supports a first electrode 38 and a second electrode 40, although in some cases there may be only a single electrode or there may be three or more electrodes. The therapy module 34 may be configured to deliver the generated therapy via the one or more electrodes 38, 40 of the electrode support 36.

The electrode support 36 may extend from the housing 24 and support one or more electrodes. In some cases, the electrode support 36 may be similar to an elongated lead with one or more electrodes mounted at locations along its length. The electrode support 36 may be flexible so that a physician can place the one or more electrodes at a desired location. In some cases, although shown schematically, the electrode support 36 may be configured to extend subcutaneously. In some cases, at least part of the electrode support 36 may be configured to extend substernal. The electrode support 36 may have a length of 1 inches, 3 inches, 4 inches, 6 inches, 8 inches, 12 inches or more, depending on the application. In some cases, the implantable device 22 may include one or more sensors 42 for sensing one or more physiological conditions of the patient.

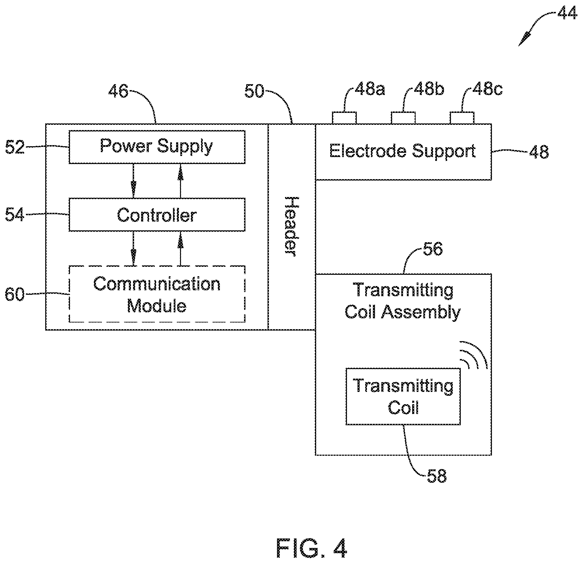

Figure 4 is a schematic block diagram of an implantable medical device (IMD) 44 that is configured to sense electrical cardiac activity, provide therapy to a patient's heart H, and provide power to a remote implantable medical device. The illustrative IMD 44 may be considered as being an example of the first IMD 12 (Figure 1). In some cases, the IMD 44 may be an implantable cardioverter-defibrillator (ICD). The IMD 44 includes a housing 46 and an electrode support 48. In some cases, the housing 46 may include a header 50, and the electrode support 48 may extend from the header 50. In the example shown, the electrode support 48 supports a plurality of electrodes 48a, 48b, 48c as illustrated, although in some cases there may be fewer than three electrodes or four or more electrodes. In some cases, the electrode support may be configured to place the electrodes 48a, 48b, 48c subcutaneously proximate a sternum of the patient and over the heart. In some instances, the electrode support 48 may be configured to place at least one of the electrodes 48a, 48b, 48c substernal. The electrode support 48 may be flexible and may be similar to a lead with the electrodes 48a, 48b, 48c supported along the length of the lead.

A power supply 52 is disposed within the housing 46. A controller 54 is disposed within the housing 46 and is operably coupled to the power supply 52 and to the electrodes 48a, 48b, 48c such that the controller 54 is configured to sense electrical cardiac activity and delivery therapy to the patient's heart via at least some of the electrodes 48a, 48b, 48c. A transmitting coil assembly 56 extends from the housing 46 and includes a transmitting coil 58 for delivering inductive power to a receive coil of a remote implantable medical device. In some cases, if the IMD 44 includes the header 50, the transmitting coil assembly 56 may extend from the header 50.

In some cases, the IMD 44 may include a communication module 60 that is operably coupled to the controller 54 so that the IMD 44 is able to receive a request for power from the remote implantable medical device. In some cases, the controller 54 may be operatively coupled to the transmitting coil 58 and may be configured to periodically, intermittently, or continuously transmit inductive power to the receive coil of the remote implantable medical device via the transmitting coil 58 in accordance with a power transmission schedule, without receiving a request for power from the remote implantable medical device.

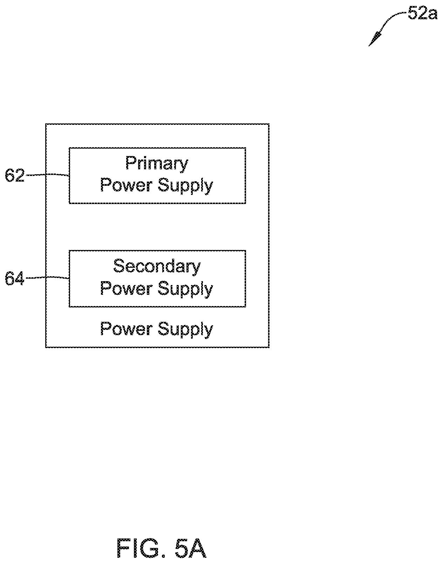

Figure 5A is a schematic illustration of a power supply 52a that may be used as the power supply 52 in the IMD 44. The power supply 52a may include a primary power supply 62 and a secondary power supply 64. In some cases, the primary power supply 62 may provide the power to delivery therapy to the patient's heart H via the electrodes 48a, 48b, 48c, but does not provide the inductive power to the receive coil of the remote implantable medical device via the transmitting coil 58. In some cases, the secondary power supply 64 provides the inductive power to the receive coil of the remote implantable medical device via the transmitting coil 58. In some cases, the power supply 52 (Figure 4) is a single power supply that supplies power both for providing therapy to the patient's heart H and to provide inductive power to the remote device, and the controller 54 monitors power consumption to ensure it has enough power for meeting critical therapy demands.

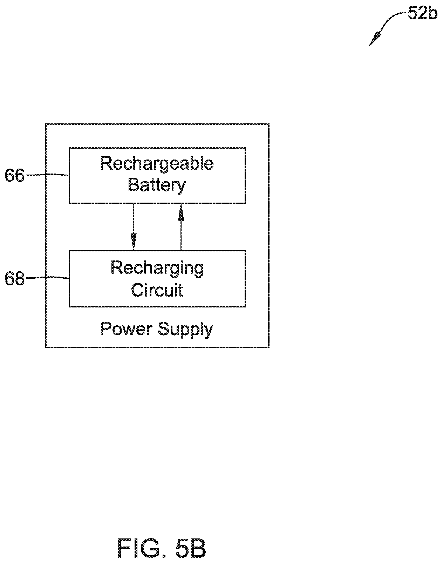

Figure 5B is a schematic illustration of a power supply 52b that may be used as the power supply 52 in the IMD 44. In some cases, for example, the power supply 52b includes a rechargeable battery 66 and a recharging circuit 68 for receiving energy transmitted to the IMD for recharging the rechargeable battery. In some cases, the recharging circuit 68 may be distinct from the controller 54. In some instances, the recharging circuit 68 may be part of the controller 54, and/or the functionality of the recharging circuit 68 may be included in the controller 54. In some cases, the recharging circuit 68 may receive energy from a device external to the patient (e.g. a device programmer) in order to recharge the rechargeable battery 66 of the IMD 44. In some cases, the energy from the rechargeable battery 66 may be used to power to the transmit coil of the IMD 44 to provide inductive power to a remote implantable medical device such as implantable device 14 of Figure 1.

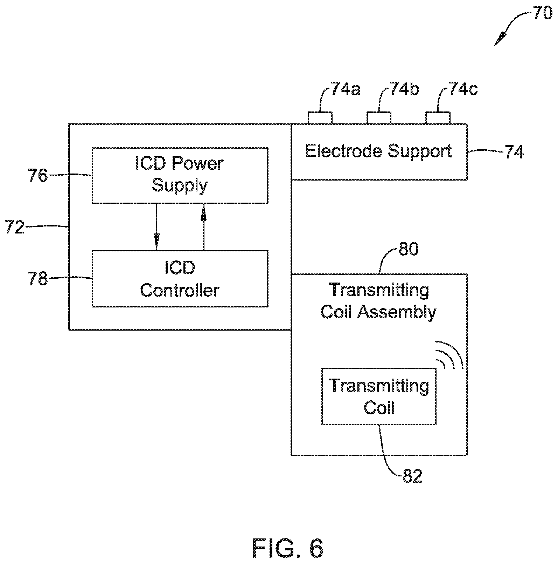

Figure 6 is a schematic block diagram of an implantable cardioverter-defibrillator (ICD) 70 that may be considered as being an example of the first IMD 12 (Figure 1) or the ICD 18 (Figure 2). The ICD 70 includes a housing 72 and an electrode support 74 extending from the housing 72. In the example shown, the electrode support 74 includes electrodes 74a, 74b, 74c, although in some cases the electrode support 74 may include fewer electrodes or even additional electrodes relative to those shown. An ICD power supply 76 is disposed within the housing 72. An ICD controller 78 is also disposed within the housing 72 and operably coupled to the ICD power supply 76 and to the electrodes 74a, 74b, 74c such that the ICD controller 78 is configured to sense electrical cardiac activity and to delivery therapy to the patient's heart H via the electrodes 74a, 74b, 74c. A transmitting coil assembly 80 is shown extending from the housing 72 and includes a transmitting coil 82 that is operably coupled to the ICD power supply 76.

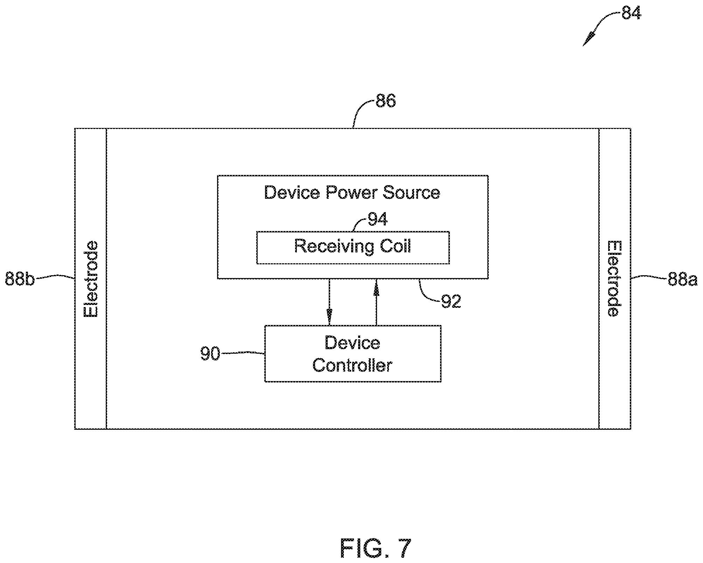

Figure 7 is a schematic block diagram of a remote implantable medical device (IMD) 84 that may be considered as being an example of the second IMD 14 (Figure 1) or the remote IMD 20 (Figure 2). The illustrative IMD 84 includes a device housing 86 and electrodes 88a, 88b secured relative to the device housing 85. While two electrodes are shown, in some cases there may be three or more electrodes present. A device controller 90 is coupled to the electrodes 88a, 88b such that the device controller 90 can sense cardiac electrical activity and deliver pacing therapy via the two or more of the electrodes 88a, 88b. A device power source 92 is coupled to the device controller 90 such that the power source 92 can power operation of the device controller 90 and to deliver pacing therapy. In some cases, as shown, the device power source 92 may include a receiving coil 94 that is configured to receive inductive power from a transmitting coil of another device such as the ICD 70 shown in Figure 6. In some cases, the device power source 92 includes a rechargeable battery. In some cases, the device power source 92 includes a power storage device such as a capacitor that stores energy provided from the transmitting coil of the ICD 70 to the receiving coil of the remote IMD 84. In some cases, the remote IMD 84 may receive energy from the receiving coil 94 for each pace that is generated by the remote IMD 84.

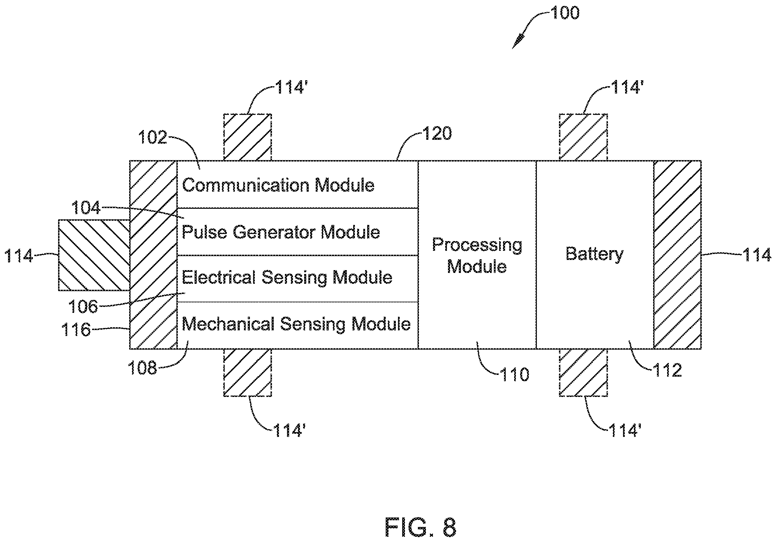

Figure 8 depicts an illustrative leadless cardiac pacemaker (LCP) that may be implanted into a patient and may operate to deliver appropriate therapy to the heart, such as to deliver anti-tachycardia pacing (ATP) therapy, cardiac resynchronization therapy (CRT), bradycardia therapy, and/or the like. As can be seen in Figure 8, the LCP 100 may be a compact device with all components housed within the or directly on a housing 120. In some cases, the LCP 100 may be considered as being an example of the first IMD 12 (Figure 1), the ICD 18 (Figure 2) or the remote IMD 84 (Figure 7). In the example shown in Figure 8, the LCP 100 may include a communication module 102, a pulse generator module 104, an electrical sensing module 106, a mechanical sensing module 108, a processing module 110, a battery 112, and an electrode arrangement 114. The LCP 100 may also include a receive coil for receiving inductive power, and a recharge circuit for recharging the battery 112 (or capacitor) using the received inductive power. It is contemplated that the LCP 100 may include more or fewer modules, depending on the application.

The communication module 102 may be configured to communicate with devices such as sensors, other medical devices such as an SICD, another LCP, and/or the like, that are located externally to the LCP 100. Such devices may be located either external or internal to the patient's body. Irrespective of the location, external devices (i.e. external to the LCP 100 but not necessarily external to the patient's body) can communicate with the LCP 100 via communication module 102 to accomplish one or more desired functions. For example, the LCP 100 may communicate information, such as sensed electrical signals, data, instructions, messages, R-wave detection markers, etc., to an external medical device (e.g. SICD and/or programmer) through the communication module 102. The external medical device may use the communicated signals, data, instructions, messages, R-wave detection markers, etc., to perform various functions, such as determining occurrences of arrhythmias, delivering electrical stimulation therapy, storing received data, and/or performing any other suitable function. The LCP 100 may additionally receive information such as signals, data, instructions and/or messages from the external medical device through the communication module 102, and the LCP 100 may use the received signals, data, instructions and/or messages to perform various functions, such as determining occurrences of arrhythmias, delivering electrical stimulation therapy, storing received data, and/or performing any other suitable function. The communication module 102 may be configured to use one or more methods for communicating with external devices. For example, the communication module 102 may communicate via radiofrequency (RF) signals, inductive coupling, optical signals, acoustic signals, conducted communication signals, and/or any other signals suitable for communication.

In the example shown in Figure 8, the pulse generator module 104 may be electrically connected to the electrodes 114. In some examples, the LCP 100 may additionally include electrodes 114'. In such examples, the pulse generator 104 may also be electrically connected to the electrodes 114'. The pulse generator module 104 may be configured to generate electrical stimulation signals. For example, the pulse generator module 104 may generate and deliver electrical stimulation signals by using energy stored in the battery 112 within the LCP 100 and deliver the generated electrical stimulation signals via the electrodes 114 and/or 114'. Alternatively, or additionally, the pulse generator 104 may include one or more capacitors, and the pulse generator 104 may charge the one or more capacitors by drawing energy from the battery 112. The pulse generator 104 may then use the energy of the one or more capacitors to deliver the generated electrical stimulation signals via the electrodes 114 and/or 114'. In at least some examples, the pulse generator 104 of the LCP 100 may include switching circuitry to selectively connect one or more of the electrodes 114 and/or 114' to the pulse generator 104 in order to select which of the electrodes 114/114' (and/or other electrodes) the pulse generator 104 delivers the electrical stimulation therapy. The pulse generator module 104 may generate and deliver electrical stimulation signals with particular features or in particular sequences in order to provide one or multiple of a number of different stimulation therapies. For example, the pulse generator module 104 may be configured to generate electrical stimulation signals to provide electrical stimulation therapy to combat bradycardia, tachycardia, cardiac synchronization, bradycardia arrhythmias, tachycardia arrhythmias, fibrillation arrhythmias, cardiac synchronization arrhythmias and/or to produce any other suitable electrical stimulation therapy. Some more common electrical stimulation therapies include anti-tachycardia pacing (ATP) therapy, cardiac resynchronization therapy (CRT), and cardioversion/defibrillation therapy. In some cases, the pulse generator 104 may provide a controllable pulse energy. In some cases, the pulse generator 104 may allow the controller to control the pulse voltage, pulse width, pulse shape or morphology, and/or any other suitable pulse characteristic.

In some examples, the LCP 100 may include an electrical sensing module 106, and in some cases, a mechanical sensing module 108. The electrical sensing module 106 may be configured to sense the cardiac electrical activity of the heart. For example, the electrical sensing module 106 may be connected to the electrodes 114/114', and the electrical sensing module 106 may be configured to receive cardiac electrical signals conducted through the electrodes 114/114'. The cardiac electrical signals may represent local information from the chamber in which the LCP 100 is implanted. For instance, if the LCP 100 is implanted within a ventricle of the heart (e.g. RV, LV), cardiac electrical signals sensed by the LCP 100 through the electrodes 114/114' may represent ventricular cardiac electrical signals. In some cases, the LCP 100 may be configured to detect cardiac electrical signals from other chambers (e.g. far field), such as the P-wave from the atrium.

The mechanical sensing module 108 may include one or more sensors, such as an accelerometer, a pressure sensor, a heart sound sensor, a blood-oxygen sensor, a chemical sensor, a temperature sensor, a flow sensor and/or any other suitable sensors that are configured to measure one or more mechanical/chemical parameters of the patient. Both the electrical sensing module 106 and the mechanical sensing module 108 may be connected to a processing module 110, which may provide signals representative of the sensed mechanical parameters. Although described with respect to Figure 8 as separate sensing modules, in some cases, the electrical sensing module 106 and the mechanical sensing module 108 may be combined into a single sensing module, as desired.

The electrodes 114/114' can be secured relative to the housing 120 but exposed to the tissue and/or blood surrounding the LCP 100. In some cases, the electrodes 114 may be generally disposed on either end of the LCP 100 and may be in electrical communication with one or more of the modules 102, 104, 106, 108, and 110. The electrodes 114/114' may be supported by the housing 120, although in some examples, the electrodes 114/114' may be connected to the housing 120 through short connecting wires such that the electrodes 114/114' are not directly secured relative to the housing 120. In examples where the LCP 100 includes one or more electrodes 114', the electrodes 114' may in some cases be disposed on the sides of the LCP 100, which may increase the number of electrodes by which the LCP 100 may sense cardiac electrical activity, deliver electrical stimulation and/or communicate with an external medical device. The electrodes 114/114' can be made up of one or more biocompatible conductive materials such as various metals or alloys that are known to be safe for implantation within a human body. In some instances, the electrodes 114/114' connected to the LCP 100 may have an insulative portion that electrically isolates the electrodes 114/114' from adjacent electrodes, the housing 120, and/or other parts of the LCP 100. In some cases, one or more of the electrodes 114/114' may be provided on a tail (not shown) that extends away from the housing 120.

The processing module 110 can be configured to control the operation of the LCP 100. For example, the processing module 110 may be configured to receive electrical signals from the electrical sensing module 106 and/or the mechanical sensing module 108. Based on the received signals, the processing module 110 may determine, for example, abnormalities in the operation of the heart H. Based on any determined abnormalities, the processing module 110 may control the pulse generator module 104 to generate and deliver electrical stimulation in accordance with one or more therapies to treat the determined abnormalities. The processing module 110 may further receive information from the communication module 102. In some examples, the processing module 110 may use such received information to help determine whether an abnormality is occurring, determine a type of abnormality, and/or to take particular action in response to the information. The processing module 110 may additionally control the communication module 102 to send/receive information to/from other devices.

In some examples, the processing module 110 may include a pre-programmed chip, such as a very-large-scale integration (VLSI) chip and/or an application specific integrated circuit (ASIC). In such embodiments, the chip may be pre-programmed with control logic in order to control the operation of the LCP 100. By using a pre-programmed chip, the processing module 110 may use less power than other programmable circuits (e.g. general purpose programmable microprocessors) while still being able to maintain basic functionality, thereby potentially increasing the battery life of the LCP 100. In other examples, the processing module 110 may include a programmable microprocessor. Such a programmable microprocessor may allow a user to modify the control logic of the LCP 100 even after implantation, thereby allowing for greater flexibility of the LCP 100 than when using a pre-programmed ASIC. In some examples, the processing module 110 may further include a memory, and the processing module 110 may store information on and read information from the memory. In other examples, the LCP 100 may include a separate memory (not shown) that is in communication with the processing module 110, such that the processing module 110 may read and write information to and from the separate memory.

The battery 112 may provide power to the LCP 100 for its operations. In some examples, the battery 112 may be a non-rechargeable lithium-based battery. In other examples, a non-rechargeable battery may be made from other suitable materials, as desired. Because the LCP 100 is an implantable device, access to the LCP 100 may be limited after implantation. Accordingly, it is desirable to have sufficient battery capacity to deliver therapy over a period of treatment such as days, weeks, months, years or even decades. In some instances, the battery 112 may a rechargeable battery, which may help increase the useable lifespan of the LCP 100. A recharge circuit may receive power from a receiving coil of the LCP 100, and use the received power to recharge the rechargeable battery. In still other examples, the battery 112 may be some other type of power source, as desired.

To implant the LCP 100 inside a patient's body, an operator (e.g., a physician, clinician, etc.), may fix the LCP 100 to the cardiac tissue of the patient's heart. To facilitate fixation, the LCP 100 may include one or more anchors 116. The anchor 116 may include any one of a number of fixation or anchoring mechanisms. For example, the anchor 116 may include one or more pins, staples, threads, screws, helix, tines, and/or the like. In some examples, although not shown, the anchor 116 may include threads on its external surface that may run along at least a partial length of the anchor 116. The threads may provide friction between the cardiac tissue and the anchor to help fix the anchor 116 within the cardiac tissue. In other examples, the anchor 116 may include other structures such as barbs, spikes, or the like to facilitate engagement with the surrounding cardiac tissue.

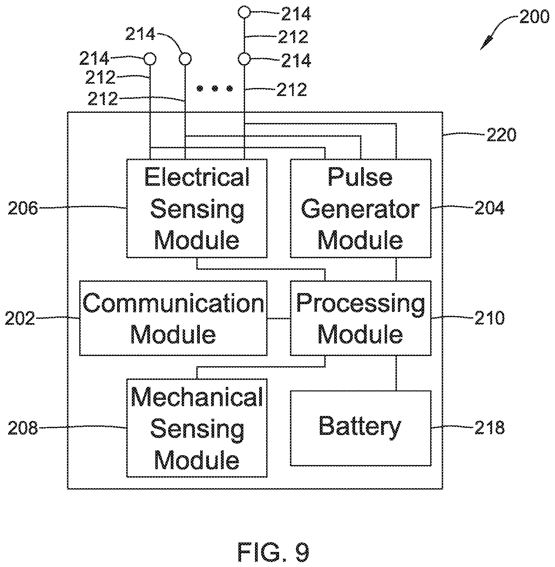

Figure 9 depicts an example of another or second medical device (MD) 200, which may be used in conjunction with the LCP 100 (Figure 8) in order to detect and/or treat cardiac abnormalities. In some cases, the MD 200 may be considered as an example of the first IMD 12 (Figure 1), the ICD 18 (Figure 2), the implantable device 22 (Figure 3), the IMD 44 (Figure 4) and the ICD 70 (Figure 6), and may for example represent an implantable cardioverter defibrillator (ICD) or a subcutaneous implantable cardioverter defibrillator (SICD). In the example shown, the MD 200 may include a communication module 202, a pulse generator module 204, an electrical sensing module 206, a mechanical sensing module 208, a processing module 210, and a battery 218. Each of these modules may be similar to the modules 102, 104, 106, 108, and 110 of LCP 100. Additionally, the battery 218 may be similar to the battery 112 of the LCP 100. In some examples, however, the MD 200 may have a larger volume within the housing 220. In such examples, the MD 200 may include a larger battery and/or a larger processing module 210 capable of handling more complex operations than the processing module 110 of the LCP 100. The MD 200 may include a transmit coil for transmitting inductive power to a remote medical device.

While it is contemplated that the MD 200 may be another leadless device such as shown in Figure 8, in some instances the MD 200 may include leads such as leads 212. The leads 212 may include electrical wires that conduct electrical signals between the electrodes 214 and one or more modules located within the housing 220. In some cases, the leads 212 may be connected to and extend away from the housing 220 of the MD 200. In some examples, the leads 212 are implanted on, within, or adjacent to a heart of a patient. The leads 212 may contain one or more electrodes 214 positioned at various locations on the leads 212, and in some cases at various distances from the housing 220. Some leads 212 may only include a single electrode 214, while other leads 212 may include multiple electrodes 214. Generally, the electrodes 214 are positioned on the leads 212 such that when the leads 212 are implanted within the patient, one or more of the electrodes 214 are positioned to perform a desired function. In some cases, the one or more of the electrodes 214 may be in contact with the patient's cardiac tissue. In some cases, the one or more of the electrodes 214 may be positioned subcutaneously and outside of the patient's heart. In some cases, the electrodes 214 may conduct intrinsically generated electrical signals to the leads 212, e.g. signals representative of intrinsic cardiac electrical activity. The leads 212 may, in turn, conduct the received electrical signals to one or more of the modules 202, 204, 206, and 208 of the MD 200. In some cases, the MD 200 may generate electrical stimulation signals, and the leads 212 may conduct the generated electrical stimulation signals to the electrodes 214. The electrodes 214 may then conduct the electrical signals and delivery the signals to the patient's heart (either directly or indirectly). In some cases, a transmit coil may be supported by the lead, such at a location along the length of the lead that is near the receive coil of a remote implantable medical device.

The mechanical sensing module 208, as with the mechanical sensing module 108, may contain or be electrically connected to one or more sensors, such as accelerometers, acoustic sensors, blood pressure sensors, heart sound sensors, blood-oxygen sensors, and/or other sensors which are configured to measure one or more mechanical/chemical parameters of the heart and/or patient. In some examples, one or more of the sensors may be located on the leads 212, but this is not required. In some examples, one or more of the sensors may be located in the housing 220.

While not required, in some examples, the MD 200 may be an implantable medical device. In such examples, the housing 220 of the MD 200 may be implanted in, for example, a transthoracic region of the patient. The housing 220 may generally include any of a number of known materials that are safe for implantation in a human body and may, when implanted, hermetically seal the various components of the MD 200 from fluids and tissues of the patient's body.

In some cases, the MD 200 may be an implantable cardiac pacemaker (ICP). In this example, the MD 200 may have one or more leads, for example the leads 212, which are implanted on or within the patient's heart. The one or more leads 212 may include one or more electrodes 214 that are in contact with cardiac tissue and/or blood of the patient's heart. The MD 200 may be configured to sense intrinsically generated cardiac electrical signals and determine, for example, one or more cardiac arrhythmias based on analysis of the sensed signals. The MD 200 may be configured to deliver CRT, ATP therapy, bradycardia therapy, and/or other therapy types via the leads 212 implanted within the heart. In some examples, the MD 200 may additionally be configured provide defibrillation therapy.

In some instances, the MD 200 may be an implantable cardioverter-defibrillator (ICD) with the ability to pace. In such examples, the MD 200 may include one or more leads implanted within a patient's heart. The MD 200 may also be configured to sense cardiac electrical signals, determine occurrences of tachyarrhythmias based on the sensed signals, and may be configured to deliver defibrillation therapy in response to determining an occurrence of a tachyarrhythmia. In other examples, the MD 200 may be a subcutaneous implantable cardioverter-defibrillator (S-ICD) with the ability to pace. In examples where the MD 200 is an S-ICD, one of the leads 212 may be a subcutaneously implanted lead. In some instances, the lead(s) may have one or more electrodes that are placed subcutaneously and outside of the chest cavity. In other examples, the lead(s) may have one or more electrodes that are placed inside of the chest cavity, such as just interior of the sternum but outside of the heart H.

In some examples, the MD 200 may not be an implantable medical device. Rather, the MD 200 may be a device external to the patient's body, and may include skin-electrodes that are placed on a patient's body. In such examples, the MD 200 may be able to sense surface electrical signals (e.g. cardiac electrical signals that are generated by the heart or electrical signals generated by a device implanted within a patient's body and conducted through the body to the skin). In such examples, the MD 200 may be configured to deliver various types of electrical stimulation therapy, including, for example, defibrillation therapy. In some cases, the MD 200 may be external to the patient's body may include a lead that extends transvenously into the heart. The lead may be used to sense and/or pace the heart. A transmit coil may be placed on the lead and adjacent to or inside of the heart.

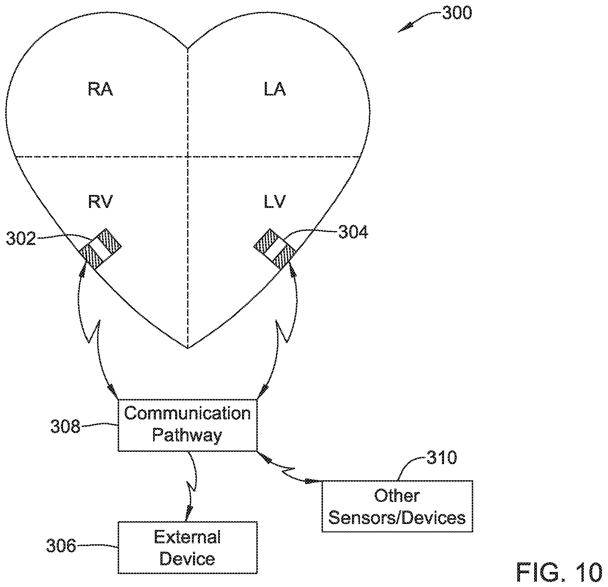

Figure 10 illustrates an example of a medical device system and a communication pathway through which multiple medical devices 302, 304, 306, and/or 310 may communicate. In the example shown, the medical device system 300 may include LCPs 302 and 304, external medical device 306, and other sensors/devices 310. The external device 306 may be any of the devices described previously with respect to the MD 200. Other sensors/devices 310 may also be any of the devices described previously with respect to the MD 200. In some instances, other sensors/devices 310 may include a sensor, such as an accelerometer, an acoustic sensor, a blood pressure sensor, or the like. In some cases, other sensors/devices 310 may include an external programmer device that may be used to program one or more devices of the system 300.

Various devices of the system 300 may communicate via communication pathway 308. The communication pathway 308 may include one or a number of different communication paths and/or a number of different communication modes. The communication pathway 308 may also include one or more distinct communication vectors. In some cases, for example, the LCPs 302 and/or 304 may sense intrinsic cardiac electrical signals and may communicate such signals to one or more other devices 302/304, 306, and 310 of the system 300 via communication pathway 308. In one example, one or more of the devices 302/304 may receive such signals and, based on the received signals, determine an occurrence of an arrhythmia. In some cases, the device or devices 302/304 may communicate such determinations to one or more other devices 306 and 310 of the system 300. In some cases, one or more of the devices 302/304, 306, and 310 of the system 300 may take action based on the communicated determination of an arrhythmia, such as by delivering a suitable electrical stimulation to the heart of the patient. It is contemplated that the communication pathway 308 may communicate using RF signals, inductive coupling, optical signals, acoustic signals, or any other signals suitable for communication. Additionally, in at least some examples, device communication pathway 308 may include multiple signal types. For instance, other sensors/device 310 may communicate with the external device 306 using a first signal type (e.g. RF communication) but communicate with the LCPs 302/304 using a second signal type (e.g. conducted communication). Further, in some examples, communication between devices may be limited. For instance, as described above, in some examples, the LCPs 302/304 may communicate with the external device 306 only through other sensors/devices 310, where the LCPs 302/304 send signals to other sensors/devices 310, and other sensors/devices 310 relay the received signals to the external device 306.

In some cases, the communication pathway 308 may include conducted communication. Accordingly, devices of the system 300 may have components that allow for such conducted communication. For instance, the devices of system 300 may be configured to transmit conducted communication signals (e.g. current and/or voltage pulses) into the patient's body via one or more electrodes of a transmitting device, and may receive the conducted communication signals (e.g. pulses) via one or more electrodes of a receiving device. The patient's body may "conduct" the conducted communication signals (e.g. pulses) from the one or more electrodes of the transmitting device to the electrodes of the receiving device in the system 300. In such examples, the delivered conducted communication signals (e.g. pulses) may differ from pacing or other therapy signals. For example, the devices of the system 300 may deliver electrical communication pulses at an amplitude /pulse width that is sub-capture threshold to the heart. Although, in some cases, the amplitude/pulse width of the delivered electrical communication pulses may be above the capture threshold of the heart, but may be delivered during a blanking period of the heart (e.g. refractory period) and/or may be incorporated in or modulated onto a pacing pulse, if desired.

Delivered electrical communication pulses may be modulated in any suitable manner to encode communicated information. In some cases, the communication pulses may be pulse width modulated or amplitude modulated. Alternatively, or in addition, the time between pulses may be modulated to encode desired information. In some cases, conducted communication pulses may be voltage pulses, current pulses, biphasic voltage pulses, biphasic current pulses, or any other suitable electrical pulse as desired.

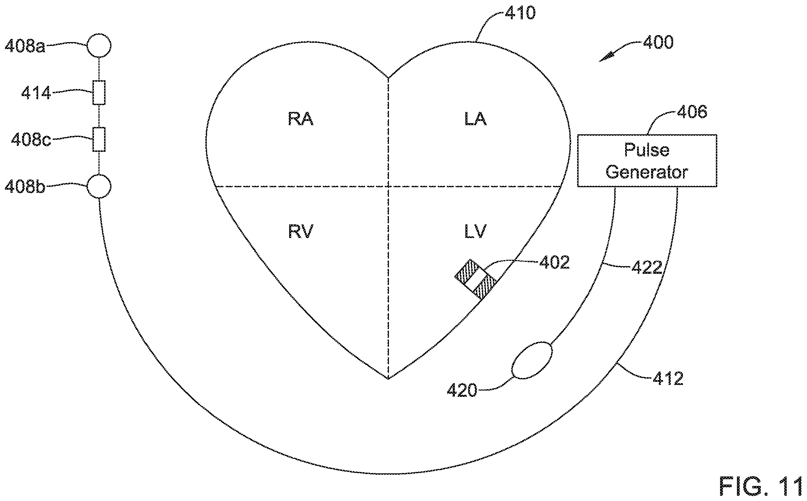

Figure 11 shows an illustrative medical device system. In Figure 11, an LCP 402 is shown fixed to the interior of the left ventricle of the heart 410, and a pulse generator 406 is shown coupled to a lead 412 having one or more electrodes 408a-408c. In some cases, the pulse generator 406 may be part of an implantable cardioverter defibrillator (ID) or a subcutaneous implantable cardioverter defibrillator (SICD), and the one or more electrodes 408a-408c may be positioned subcutaneously. The lead 412 may be flexible so that a physician can place the one or more electrodes at a desired location. The lead may have a length of 1 inches, 3 inches, 4 inches, 6 inches, 8 inches, 12 inches or more, depending on the application. In some cases, the physician may place one or more of the electrodes 408a-408c subcutaneously outside of the chest cavity but adjacent the heart. In some cases, the physician may place one or more of the electrodes 408a-408c inside of the chest cavity but outside of the heart, such as just interior of the sternum.

A transmit coil 420 may be tethered to the pulse generator 406 by a transmitting coil assembly 422. The transmitting coil assembly 422 may be flexible so that a physician can place the transmit coil 420 at a desired location, such as a substernal location adjacent the LCP 402. The transmitting coil assembly 422 may have a length of 2.54 cm (1 inch), 7.62 cm (3 inches), 10.16 cm (4 inches), 15.24 cm (6 inches), 20.32 cm (8 inches), 30.48 cm (12 inches) or more depending on the application.

In some cases, the LCP 402 may be in the right ventricle, right atrium, left ventricle or left atrium of the heart, as desired. In some cases, more than one LCP 402 may be implanted. For example, one LCP may be implanted in the right ventricle and another may be implanted in the right atrium. In another example, one LCP may be implanted in the right ventricle and another may be implanted in the left ventricle. In yet another example, one LCP may be implanted in each of the chambers of the heart. In some cases, the LCP 402 may communicate with the pulse generator 406, which in some cases may be a subcutaneous implantable cardioverter-defibrillator (SICD). In some cases, the lead 412 and/or pulse generator 406 may include an accelerometer 414 that may, for example, be configured to sense vibrations that may be indicative of heart sounds.

## Claims

1. An implantable device (12, 18, 22, 44, 70) comprising:
a housing (24, 46, 72) configured to be implantable at a subcutaneous implant location in a patient;
a power supply (26, 52, 76) disposed within the housing;
a transmitting coil assembly (28, 56, 80) extending from the housing (24, 46, 72), the transmitting coil assembly (28, 56, 80) configured to extend from the subcutaneous implant location of the housing (24, 46, 72) to a substernal location, the transmit coil assembly (28, 56, 80) including a transmit coil (30, 58, 82); and
a controller (32, 54, 78) operably coupled to the power supply (26, 52, 76) and the transmit coil (30, 58, 82), the controller (32, 54, 78) configured to selectively activate the transmit coil (30, 58, 82) to provide inductive power to a receive coil (94) of a remote medical device (14, 20, 84) implanted substernally.

2. The implantable device (12, 18, 22, 44, 70) of claim 1, further comprising a therapy module (34) for generating a therapy.

3. The implantable device (12, 18, 22, 44, 70) of claim 2, further comprising an electrode support (48, 74) extending from the housing (24, 46, 72) where the electrode support (48, 74) supports one or more electrodes (48a, 48b, 48c, 74a, 74b, 74c), further wherein the therapy module (34) is configured to deliver the generated therapy via the one or more electrodes (48a, 48b, 48c, 74a, 74b, 74c) of the electrode support (48, 74).

4. The implantable device (12, 18, 22, 44, 70) of any one of claims 1 to 3, wherein the implantable device (12, 18, 22, 44, 70) comprises one or more sensors (42) for sensing one or more physiological conditions of the patient.

5. The implantable device (12, 18, 22, 44, 70) of any one of claims 1 to 4, wherein the implantable device comprises an implantable cardioverter-defibrillator, ICD.

6. An implantable medical device, IMD, (12, 18, 22, 44, 70) configured to sense electrical cardiac activity and to provide therapy to a patient's heart and to provide power to a remote implantable medical device (14, 20, 84), the implantable medical device (12, 18, 22, 44, 70) comprising:
a housing (24, 46, 72);
an electrode support (48, 74) extending from the housing (24, 46, 72), wherein the electrode support (48, 74) supports a plurality of electrodes (48a, 48b, 48c, 74a, 74b, 74c);
a power supply (26, 52, 76) disposed within the housing (24, 46, 72);
a controller (32, 54, 78) disposed within the housing (24, 46, 72) and operably coupled to the power supply (26, 52, 76) and to the plurality of electrodes (48a, 48b, 48c, 74a, 74b, 74c) such that the controller (32, 54, 78) is configured to sense electrical cardiac activity and to deliver therapy to the patient's heart via the plurality of electrodes (48a, 48b, 48c, 74a, 74b, 74c);
a transmitting coil assembly (28, 56, 80) extending from the housing (24, 46, 72), the transmitting coil assembly (28, 56, 80) including a transmitting coil (30, 58, 82) for delivering inductive power to a receive coil of the remote implantable medical device (14, 20, 84); wherein the power supply (52a) comprises a primary power supply (62) and a secondary power supply (64), wherein the primary power supply (62) provides power to the delivery of therapy to the patient's heart via the plurality of electrodes (48a, 48b, 48c, 74a, 74b, 74c) and does not provide the inductive power to the receive coil (94) of the remote implantable medical device (14, 20, 84), and wherein the secondary power supply (64) provides the inductive power to the receive coil (94) of the remote implantable medical device (14, 20, 84).

7. The IMD (12, 18, 22, 44, 70) of claim 6, wherein the housing (46) comprises a header (50), wherein the electrode support (48) and the transmitting coil assembly (56) extend from the header (50).

8. The IMD (12, 18, 22, 44, 70) of any one of claims 6 to 7, further comprising a communication module (60) operably coupled to the controller (54) to receive a request for power from the remote implantable medical device (14, 20, 84).

9. The IMD (12, 18, 22, 44, 70) of any one of claims 6 to 8, wherein the controller (32, 54, 78) is operatively coupled to the transmitting coil (30, 58, 82), and is configured to periodically transmit indicative power to the receive coil (94) of the remote implantable medical device (14, 20, 84) via the transmitting coil (30, 58, 82) in accordance with a power transmission schedule, without receiving a request for power from the remote implantable medical device (14, 20, 84).

10. The IMD (12, 18, 22, 44, 70) of any one of claims 6 to 9, wherein the electrode support (48, 74) is configured to place the plurality of electrodes (48a, 48b, 48c, 74a, 74b, 74c) subcutaneously proximate a sternum of the patient.

11. The IMD (12, 18, 22, 44, 70) of any one of claims 6 to 9, wherein the electrode support (48, 74) is configured to place at least one of the plurality of electrodes (48a, 48b, 48c, 74a, 74b, 74c) substernally.

12. The IMD (12, 18, 22, 44, 70) of any one of claims 6 to 11, wherein the power supply (52b) comprises a rechargeable battery (66), and the IMD (12, 18, 22, 44, 70) further comprises a recharging circuit (68) for receiving energy transmitted to the IMD (12, 18, 22, 44, 70) for recharging the rechargeable battery (66).

13. A system (300, 400) for sensing electrical cardiac activity and adapted to provide therapy to a patient's heart, the system comprising:
an implantable cardioverter-defibrillator, ICD, (22, 44, 70) comprising:
a housing (24, 46, 72);
an electrode support (48, 74) extending from the housing (24, 46, 72) with a plurality of electrodes (48a, 48b, 48c, 74a, 74b, 74c) supported by the electrode support (48, 74);
an ICD power supply (26, 52, 76) disposed within the housing (24, 46, 72);
an ICD controller (32, 54, 78) disposed within the housing (24, 46, 72) and operably coupled to the ICD power supply (26, 52, 76) and to the plurality of electrodes (48a, 48b, 48c, 74a, 74b, 74c) such that the ICD controller (32, 54, 78) is configured to sense electrical cardiac activity and to deliver therapy to the patient's heart via the plurality of electrodes (48a, 48b, 48c, 74a, 74b, 74c); and
a transmitting coil assembly (28, 56, 80) extending from the housing (24, 46, 72) with a transmitting coil (30, 58, 82) operably coupled to the ICD power supply (26, 52, 76); and
a remote implantable medical device (14, 20, 84) comprising:
a device housing (86);
at least two electrodes (88a, 88b) secured relative to the device housing (86);
a device controller (90) coupled to the at least two electrodes (88a, 88b) such that the device controller (90) can sense cardiac electrical activity and deliver pacing therapy via the at least two electrodes (88a, 88b); and
a power source (92) coupled to the device controller (90) such that the power source (92) can power operation of the device controller (90) and to deliver pacing therapy, the power source (92) including a receiving coil (94) configured to receive power from the transmitting coil (30, 58, 82) of the ICD (22, 44, 70).

14. The system of claim 13, wherein the power source (92) of the remote implantable medical device (14, 20, 84) comprises a rechargeable battery or a capacitor that stores energy provided from the transmitting coil (30, 58, 82) of the ICD (22, 44, 70) to the receiving coil (94) of the remote implantable medical device (14, 20, 84).

## Patentansprüche

1. Implantierbare Vorrichtung (12, 18, 22, 44, 70), umfassend:
ein Gehäuse (24, 46, 72), dazu ausgelegt, an einem subkutanen Implantatort in einem Patienten implantierbar zu sein;
eine Stromversorgung (26, 52, 76), angeordnet innerhalb des Gehäuses;
eine Sendespulenanordnung (28, 56, 80), die sich von dem Gehäuse (24, 46, 72) erstreckt, wobei die Sendespulenanordnung (28, 56, 80) dazu ausgelegt ist, sich von dem subkutanen Implantatort des Gehäuses (24, 46, 72) zu einem substernalen Ort zu erstrecken, wobei die Sendespulenanordnung (28, 56, 80) eine Sendespule (30, 58, 82) umfasst; und
eine Steuerung (32, 54, 78), betreibbar gekoppelt an die Stromversorgung (26, 52, 76) und die Sendespule (30, 58, 82), wobei die Steuerung (32, 54, 78) dazu ausgelegt ist, die Sendespule (30, 58, 82) wahlweise zu aktivieren, um einer Empfangsspule (94) einer entfernten medizinischen Vorrichtung (14, 20, 84), substernal implantiert, induktive Energie bereitzustellen.

2. Implantierbare Vorrichtung (12, 18, 22, 44, 70) nach Anspruch 1, ferner umfassend ein Therapiemodul (34) zum Erzeugen einer Therapie.

3. Implantierbare Vorrichtung (12, 18, 22, 44, 70) nach Anspruch 2, ferner umfassend eine sich vom Gehäuse (24, 46, 72) erstreckende Elektrodenstütze (48, 74), wobei die Elektrodenstütze (48, 74) eine oder mehrere Elektroden (48a, 48b, 48c, 74a, 74b, 74c) stützt, wobei das Therapiemodul (34) ferner dazu ausgelegt ist, die generierte Therapie über eine oder mehrere Elektroden (48a, 48b, 48c, 74a, 74b, 74c) der Elektrodenstütze (48, 74) zuzuführen.

4. Implantierbare Vorrichtung (12, 18, 22, 44, 70) nach einem der Ansprüche 1 bis 3, wobei die implantierbare Vorrichtung (12, 18, 22, 44, 70) einen oder mehrere Sensoren (42) zur Erfassung eines oder mehrerer physiologischer Zustände des Patienten umfasst.

5. Implantierbare Vorrichtung (12, 18, 22, 44, 70) nach einem der Ansprüche 1 bis 4, wobei die implantierbare Vorrichtung einen implantierbaren Kardioverter-Defibrillator (ICD) umfasst.

6. Implantierbare medizinische Vorrichtung, IMD (12, 18, 22, 44, 70), dazu ausgelegt, elektrische kardiale Aktivität zu erfassen und dem Herzen eines Patienten Therapie bereitzustellen und einer entfernten implantierbaren medizinischen Vorrichtung (14, 20, 84) Energie bereitzustellen, wobei die implantierbare medizinische Vorrichtung (12, 18, 22, 44, 70) umfasst:
ein Gehäuse (24, 46, 72);
eine Elektrodenstütze (48, 74), die sich vom Gehäuse (24, 46, 72) erstreckt, wobei die Elektrodenstütze (48, 74) eine Vielzahl von Elektroden (48a, 48b, 48c, 74a, 74b, 74c) stützt;
eine Stromversorgung (26, 52, 76), angeordnet innerhalb des Gehäuses (24, 46, 72);
eine Steuerung (32, 54, 78), angeordnet innerhalb des Gehäuses (24, 46, 72) und betreibbar gekoppelt mit der Stromversorgung (26, 52, 76) und mit der Vielzahl von Elektroden (48a, 48b, 48c, 74a, 74b, 74c), sodass die Steuerung (32, 54, 78) dazu ausgelegt ist, elektrische kardiale Aktivität zu erfassen und über die Vielzahl von Elektroden (48a, 48b, 48c, 74a, 74b, 74c) dem Herzen des Patienten Therapie zuzuführen;
eine Sendespulenanordnung (28, 56, 80), die sich von dem Gehäuse (24, 46, 72) erstreckt, wobei die Sendespulenanordnung (28, 56, 80) eine Sendespule (30, 58, 82) zum Zuführen induktiver Energie zu einer Empfangsspule der entfernten implantierbaren medizinischen Vorrichtung (14, 20, 84) umfasst;
wobei die Stromversorgung (52a) eine primäre Stromversorgung (62) und eine sekundäre Stromversorgung (64) umfasst, wobei die primäre Stromversorgung (62) über die Vielzahl von Elektroden (48a, 48b, 48c, 74a, 74b, 74c) Energie zum Zuführen von Therapie zum Herzen des Patienten bereitstellt und die induktive Energie nicht der Empfangsspule (94) der entfernten implantierbaren medizinischen Vorrichtung (14, 20, 84) bereitstellt, und wobei
die sekundäre Stromversorgung (64) die induktive Energie der Empfangsspule (94) der entfernten implantierbaren medizinischen Vorrichtung (14, 20, 84) bereitstellt.

7. IMD (12, 18, 22, 44, 70) nach Anspruch 6, wobei das Gehäuse (46) einen Sockel (50) umfasst, wobei sich die Elektrodenstütze (48) und die Sendespulenanordnung (56) von dem Sockel (50) erstrecken.

8. IMD (12, 18, 22, 44, 70) nach einem der Ansprüche 6 bis 7, ferner umfassend ein Kommunikationsmodul (60), das betreibbar an die Steuerung (54) gekoppelt ist, um von der entfernten implantierbaren medizinischen Vorrichtung (14, 20, 84) eine Anforderung von Energie zu empfangen.

9. IMD (12, 18, 22, 44, 70) nach einem der Ansprüche 6 bis 8, wobei die Steuerung (32, 54, 78) operativ mit der Sendespule (30, 58, 82) gekoppelt ist und dazu ausgelegt ist, periodisch indikative Energie an die Empfangsspule (94) der entfernten implantierbaren medizinischen Vorrichtung (14, 20, 84) über die Sendespule (30, 58, 82) entsprechend einem Energieübertragungsplan zu übertragen, ohne von der entfernten implantierbaren medizinischen Vorrichtung (14, 20, 84) eine Anforderung von Energie zu empfangen.

10. IMD (12, 18, 22, 44, 70) nach einem der Ansprüche 6 bis 9, wobei die Elektrodenstütze (48, 74) dazu ausgelegt ist, die Vielzahl von Elektroden (48a, 48b, 48c, 74a, 74b, 74c) subkutan in der Nähe eines Sternums des Patienten zu platzieren.

11. IMD (12, 18, 22, 44, 70) nach einem der Ansprüche 6 bis 9, wobei die elektronische Stütze (48, 74) dazu ausgelegt ist, mindestens eine von der Vielzahl von Elektroden (48a, 48b, 48c, 74a, 74b, 74c) substernal zu platzieren.

12. IMD (12, 18, 22, 44, 70) nach einem der Ansprüche 6 bis 11, wobei die Stromversorgung (52b) eine aufladbare Batterie (66) umfasst, und wobei die IMD (12, 18, 22, 44, 70) ferner eine Ladeschaltung (68) zum Empfangen von an die IMD (12, 18, 22, 44, 70) übertragener Energie zum Aufladen der aufladbaren Batterie (66) umfasst.

13. System (300, 400) zur Erfassung elektrischer kardialer Aktivität und dazu angepasst, dem Herzen eines Patienten Therapie bereitzustellen, wobei das System umfasst:
einen implantierbaren Kardioverter-Defibrillator, ICD (22, 44, 70), umfassend:
ein Gehäuse (24, 46, 72);
eine Elektrodenstütze (48, 74), die sich von dem Gehäuse (24, 46, 72) erstreckt, mit einer Vielzahl von Elektroden (48a, 48b, 48c, 74a, 74b, 74c), die von der Elektrodenstütze (48, 74) gestützt werden;
eine ICD-Stromversorgung (26, 52, 76), angeordnet innerhalb des Gehäuses (24, 46, 72);
eine ICD-Steuerung (32, 54, 78), angeordnet innerhalb des Gehäuses (24, 46, 72) und betreibbar gekoppelt mit der ICD-Stromversorgung (26, 52, 76) und mit der Vielzahl von Elektroden (48a, 48b, 48c, 74a, 74b, 74c), sodass die ICD-Steuerung (32, 54, 78) dazu ausgelegt ist, elektrische kardiale Aktivität zu erfassen und über die Vielzahl von Elektroden (48a, 48b, 48c, 74a, 74b, 74c) dem Herzen des Patienten Therapie zuzuführen; und
eine sich von dem Gehäuse (24, 46, 72) erstreckende Sendespulenanordnung (28, 56, 80) mit einer Sendespule (30, 58, 82), die betreibbar an die ICD-Stromversorgung (26, 52, 76) gekoppelt ist;
und eine entfernte implantierbare medizinische Vorrichtung (14, 20, 84), umfassend:
ein Vorrichtungsgehäuse (86);
mindestens zwei Elektroden (88a, 88b), relativ zu dem Vorrichtungsgehäuse (86) gesichert;
eine Vorrichtungssteuerung (90), gekoppelt an die mindestens zwei Elektroden (88a, 88b), sodass die Vorrichtungssteuerung (90) elektrische kardiale Aktivität erfassen und über die mindestens zwei Elektroden (88a, 88b) Stimulationstherapie zuführen kann; und
eine Energiequelle (92), gekoppelt an die Vorrichtungssteuerung (90), sodass die Energiequelle (92) einen Betrieb der Vorrichtungssteuerung (90) mit Energie versorgen und Stimulationstherapie zuführen kann, wobei die Energiequelle (92) eine Empfangsspule (94) umfasst, die dazu ausgelegt ist, Energie von der Sendespule (30, 58, 82) der ICD (22, 44, 70) zu empfangen.

14. System nach Anspruch 13, wobei die Energiequelle (92) der entfernten implantierbaren medizinischen Vorrichtung (14, 20, 84) eine aufladbare Batterie oder einen Kondensator umfasst, die/der von der Sendespule (30, 58, 82) der ICD (22, 44, 70) für die Empfangsspule (94) der entfernten medizinischen Vorrichtung (14, 20, 84) bereitgestellte Energie speichert.

## Revendications

1. Dispositif implantable (12, 18, 22, 44, 70) comprenant :
un boîtier (24, 46, 72) qui est configuré de manière à ce qu'il puisse être implanté au niveau d'une localisation d'implant sous-cutanée chez un patient ;
une alimentation en énergie électrique (26, 52, 76) qui est disposée à l'intérieur du boîtier ;
un assemblage de bobine d'émission (28, 56, 80) qui s'étend depuis le boîtier (24, 46, 72), l'assemblage de bobine d'émission (28, 56, 80) étant configuré de manière à ce qu'il s'étende depuis la localisation d'implant sous-cutanée du boîtier (24, 46, 72) jusqu'à une localisation infrasternale, l'assemblage de bobine d'émission (28, 56, 80) incluant une bobine d'émission (30, 58, 82) ; et
un contrôleur (32, 54, 78) qui est couplé de manière opérationnelle à l'alimentation en énergie électrique (26, 52, 76) et à la bobine d'émission (30, 58, 82), le contrôleur (32, 54, 78) étant configuré de manière à ce qu'il active de façon sélective la bobine d'émission (30, 58, 82) afin de fournir de l'énergie électrique inductive à une bobine de réception (94) d'un dispositif médical à distance (14, 20, 84) qui est implanté de façon infrasternale.

2. Dispositif implantable (12, 18, 22, 44, 70) selon la revendication 1, comprenant en outre un module de thérapie (34) pour générer une thérapie.

3. Dispositif implantable (12, 18, 22, 44, 70) selon la revendication 2, comprenant en outre un support d'électrodes(s) (48, 74) qui s'étend depuis le boîtier (24, 46, 72), dans lequel le support d'électrodes(s) (48, 74) supporte une ou plusieurs électrode(s) (48a, 48b, 48c, 74a, 74b, 74c), en outre dans lequel le module de thérapie (34) est configuré pour administrer la thérapie générée via les une ou plusieurs électrodes (48a, 48b, 48c, 74a, 74b, 74c) du support d'électrode(s) (48, 74).

4. Dispositif implantable (12, 18, 22, 44, 70) selon l'une quelconque des revendications 1 à 3, dans lequel le dispositif implantable (12, 18, 22, 44, 70) comprend un ou plusieurs capteur(s) (42) pour détecter une ou plusieurs condition(s) physiologique(s) du patient.

5. Dispositif implantable (12, 18, 22, 44, 70) selon l'une quelconque des revendications 1 à 4, dans lequel le dispositif implantable comprend un dispositif de cardioversion - défibrillateur implantable, ICD.

6. Dispositif médical implantable, IMD, (12, 18, 22, 44, 70) configuré pour détecter une activité cardiaque électrique et pour administrer une thérapie au cœur d'un patient ainsi que pour fournir de l'énergie électrique à un dispositif médical implantable à distance (14, 20, 84), le dispositif médical implantable (12, 18, 22, 44, 70) comprenant :
un boîtier (24, 46, 72) ;
un support d'électrodes (48, 74) qui s'étend depuis le boîtier (24, 46, 72), dans lequel le support d'électrodes (48, 74) supporte une pluralité d'électrodes (48a, 48b, 48c, 74a, 74b, 74c) ;
une alimentation en énergie électrique (26, 52, 76) qui est disposée à l'intérieur du boîtier (24, 46, 72) ;
un contrôleur (32, 54, 78) qui est disposé à l'intérieur du boîtier (24, 46, 72) et qui est couplé de manière opérationnelle à l'alimentation en énergie électrique (26, 52, 76) et aux électrodes de la pluralité d'électrodes (48a, 48b, 48c, 74a, 74b, 74c) de telle sorte que le contrôleur (32, 54, 78) soit configuré pour détecter l'activité cardiaque électrique et pour administrer une thérapie au cœur du patient via les électrodes de la pluralité d'électrodes (48a, 48b, 48c, 74a, 74b, 74c) ;
un assemblage de bobine d'émission (28, 56, 80) qui s'étend depuis le boîtier (24, 46, 72), l'assemblage de bobine d'émission (28, 56, 80) incluant une bobine d'émission (30, 58, 82) pour délivrer de l'énergie électrique inductive à une bobine de réception du dispositif médical implantable à distance (14, 20, 84) ;
dans lequel l'alimentation en énergie électrique (52a) comprend une alimentation en énergie électrique de primaire (62) et une alimentation en énergie électrique de secondaire (64), dans lequel l'alimentation en énergie électrique de primaire (62) fournit de l'énergie électrique pour l'administration de la thérapie au cœur du patient via les électrodes de la pluralité d'électrodes (48a, 48b, 48c, 74a, 74b, 74c) et ne fournit pas de l'énergie électrique inductive à la bobine de réception (94) du dispositif médical implantable à distance (14, 20, 84) ; et
dans lequel l'alimentation en énergie électrique de secondaire (64) fournit de l'énergie électrique inductive à la bobine de réception (94) du dispositif médical implantable à distance (14, 20, 84).

7. IMD (12, 18, 22, 44, 70) selon la revendication 6, dans lequel le boîtier (46) comprend une partie de tête (50), dans lequel le support d'électrodes (48) et l'assemblage de bobine d'émission (56) s'étendent depuis la partie de tête (50).

8. IMD (12, 18, 22, 44, 70) selon l'une quelconque des revendications 6 et 7, comprenant en outre un module de communication (60) qui est couplé de manière opérationnelle au contrôleur (54) pour recevoir une requête demandant de l'énergie électrique en provenance du dispositif médical implantable à distance (14, 20, 84).

9. IMD (12, 18, 22, 44, 70) selon l'une quelconque des revendications 6 à 8, dans lequel le contrôleur (32, 54, 78) est couplé de manière opérationnelle à la bobine d'émission (30, 58, 82) et est configuré pour transmettre de façon périodique de l'énergie électrique inductive à la bobine de réception (94) du dispositif médical implantable à distance (14, 20, 84) via la bobine d'émission (30, 58, 82) conformément à une planification de transmission d'énergie électrique, sans la réception d'une requête demandant de l'énergie électrique en provenance du dispositif médical implantable à distance (14, 20, 84).

10. IMD (12, 18, 22, 44, 70) selon l'une quelconque des revendications 6 à 9, dans lequel le support d'électrodes (48, 74) est configuré pour positionner les électrodes de la pluralité d'électrodes (48a, 48b, 48c, 74a, 74b, 74c) de façon soucutanée à proximité du sternum du patient.

11. IMD (12, 18, 22, 44, 70) selon l'une quelconque des revendications 6 à 9, dans lequel le support d'électrodes (48, 74) est configuré pour positionner au moins l'une de la pluralité d'électrodes (48a, 48b, 48c, 74a, 74b, 74c) de façon infrasternale.

12. IMD (12, 18, 22, 44, 70) selon l'une quelconque des revendications 6 à 11, dans lequel l'alimentation en énergie électrique (52b) comprend une batterie rechargeable (66), et l'IMD (12, 18, 22, 44, 70) comprend en outre un circuit de rechargement (68) pour recevoir l'énergie électrique qui est transmise à l'IMD (12, 18, 22, 44, 70) pour recharger la batterie rechargeable (66).

13. Système (300, 400) pour détecter l'activité cardiaque électrique et adapté pour administrer une thérapie au cœur d'un patient, le système comprenant :
un dispositif de cardioversion - défibrillateur implantable, ICD, (22, 44, 70) qui comprend :
un boîtier (24, 46, 72) ;
un support d'électrodes (48, 74) qui s'étend depuis le boîtier (24, 46, 72), les électrodes d'une pluralité d'électrodes (48a, 48b, 48c, 74a, 74b, 74c) étant supportées par le support d'électrodes (48, 74);
une alimentation en énergie électrique d'ICD (26, 52, 76) qui est disposée à l'intérieur du boîtier (24, 46, 72) ;
un contrôleur d'ICD (32, 54, 78) qui est disposé à l'intérieur du boîtier (24, 46, 72) et qui est couplé de manière opérationnelle à l'alimentation en énergie électrique d'ICD (26, 52, 76) et aux électrodes de la pluralité d'électrodes (48a, 48b, 48c, 74a, 74b, 74c) de telle sorte que le contrôleur d'ICD (32, 54, 78) soit configuré pour détecter l'activité cardiaque électrique et pour administrer une thérapie au cœur du patient via les électrodes de la pluralité d'électrodes (48a, 48b, 48c, 74a, 74b, 74c) ; et
un assemblage de bobine d'émission (28, 56, 80) qui s'étend depuis le boîtier (24, 46, 72) et qui comprend une bobine d'émission (30, 58, 82) qui est couplée de manière opérationnelle à l'alimentation en énergie électrique d'ICD (26, 52, 76) ;
un dispositif médical implantable à distance (14, 20, 84) qui comprend :
un boîtier de dispositif (86) ;
au moins deux électrodes (88a, 88b) qui sont fixées par rapport au boîtier de dispositif (86) ;
un contrôleur de dispositif (90) qui est couplé aux au moins deux électrodes (88a, 88b) de telle sorte que le contrôleur de dispositif (90) puisse détecter l'activité électrique cardiaque et administrer une thérapie de stimulation cardiaque via les au moins deux électrodes (88a, 88b) ; et
une source d'énergie électrique (92) qui est couplée au contrôleur de dispositif (90) de telle sorte que la source d'énergie électrique (92) puisse alimenter en énergie électrique le contrôleur de dispositif (90) ainsi qu'administrer une thérapie de stimulation cardiaque, la source d'énergie électrique (92) incluant une bobine de réception (94) qui est configurée pour recevoir l'énergie électrique en provenance de la bobine d'émission (30, 58, 82) de l'ICD (22, 44, 70).

14. Système selon la revendication 13, dans lequel la source d'énergie électrique (92) du dispositif médical implantable à distance (14, 20, 84) comprend une batterie rechargeable ou un condensateur qui stocke l'énergie électrique qui est fournie depuis la bobine d'émission (30, 58, 82) de l'ICD (22, 44, 70) à la bobine de réception (94) du dispositif médical implantable à distance (14, 20, 84).
